# EUROPEAN PATENT SPECIFICATION

(11) **EP 3 166 557 B1**
(45) Date of publication and mention of the grant of the patent: **25.10.2017**
(21) Application number: 15753179.9
(22) Date of filing: 01.07.2015
(51) Int. Cl.: A61G 13/10

(54) **SUPPORT DEVICE FOR PERCUTANEOUS INTERVENTIONS**
UNTERSTÜTZUNGSVORRICHTUNG FÜR PERKUTANE EINGRIFFE
DISPOSITIF DE SUPPORT POUR INTERVENTIONS PERCUTANÉES

(30) Priority: 09.07.2014 IT RM20140371
(43) Date of publication of application: 17.05.2017
(73) Proprietor: Neri, Roberto, 00062 Bracciano (RM) (IT)
(72) Inventor: Neri, Roberto, 00062 Bracciano (RM) (IT)
(74) Representative: Papa, Elisabetta
(86) International application number: PCT/IB2015/054963
(87) International publication number: WO 2016/005859

(56) References cited:
- WO-A1-2007/048323
- US-A1- 2010 139 005
- US-A1- 2013 318 714

## Description

### Field of the invention

The present invention relates to a support device, in particular to a support device for a medical device, suitable to support the surgical instrumentation during percutaneous interventional operations.

### Background of the invention

Percutaneous interventional operations have had in recent years an exponential growth in number and complexity of the interventions made.

Medical devices most commonly used during percutaneous interventional operations, against a length of 1 to 3 meters, often have a sub-millimetric caliber. For this reason they require fine manipulations for precise positioning and to avoid damage to their delicate structure.

US 2013/0318714 discloses a surgical station which includes a patient support base and an operation platform. Currently medical devices are often maintained suspended by the operator assisted by one or more assistants or placed in conditions of precarious equilibrium on the sterile drapes covering the patient.

The need to support the medical devices during the operation, therefore, limits the freedom to handling both of the operator and the assistants during the intervention performed.

In addition, the practice adopted of resting precariously medical devices on drapes can lead to unwanted overlapping and/or interlacing of the devices that, having to be reported to proper positioning, involve unwanted delays in the timing of the intervention carried out.

One additional disadvantage is represented by the radiological exposure of the operators during procedures that require the use of X-rays. In such circumstances, the operators are exposed to the direct and diffuse radiations, especially in parts of the body not covered by the radiation protection wearable devices, such as hands.

### Summary of the invention

The technical problem posed and solved by the present invention is that of providing a support device, in particular for supporting medical devices during percutaneous interventional operations, allowing overcoming the drawbacks mentioned above with reference to the known art.

This problem is solved by a device according to claim 1.

Preferred features of the present invention are provided in the dependent claims.

The present invention provides some relevant advantages.

The main advantage consists in the fact that the support device according to the invention allows the operator to position the medical device in an orderly and more favorable way for performing the procedure. Furthermore, the invention allows operators to easily manipulate these devices without incurring in unwanted tangling and without the possibility of impact with the patient body.

Advantageously, such a device is adjustable so as to better adapt to the specific physical conformation of the patient.

The device according to the invention, especially by virtue of the realizability of its main body in plastic material, is light and of reduced cost. Another advantage consists in the fact that a preferred embodiment of the present invention allows the use a large work surface, flat and rigid, resting on the angiographic table and partly positioned above the patient.

Advantageously, the support device according to the present invention can be realized in X-ray shielding materials and used during operations wherein there is a simultaneous X-rays irradiation, this entailing a significant reduction of the radiological exposure of body parts of the operators, such as hands, currently poorly protectable.

Other advantages, features and use modes of the present invention will result evident from the following detailed description of some embodiments, shown by way of example and not with limitative purpose.

### Brief description of the drawings

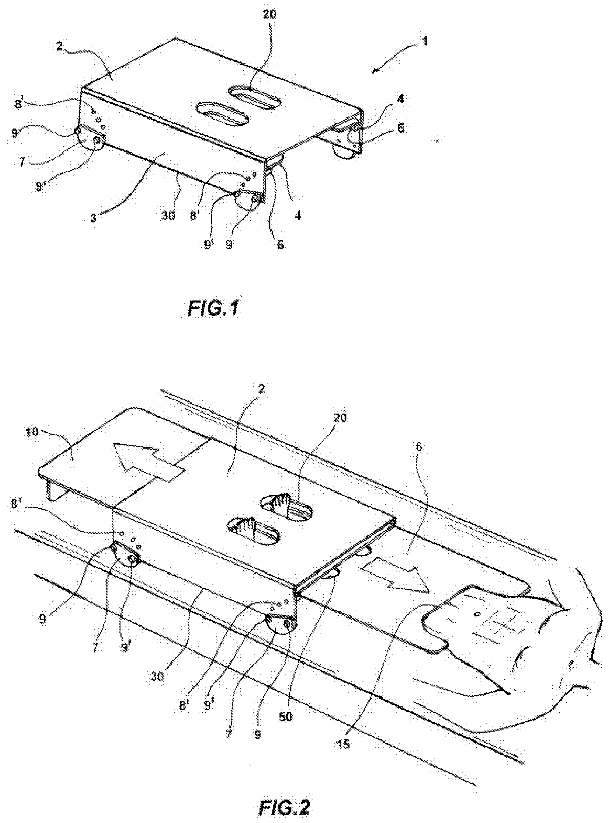

Reference to the figures of the enclosed drawings will be made, wherein:
▪ Figure 1 shows a prospective view of a first preferred embodiment of the support device according to the present invention, in a non-use configuration; and
▪ Figure 2 shows a further prospective view of the device of Figure 1 in a use configuration.

### Detailed description of preferred embodiments of the invention

By firstly referring to Figure 1, a support device according to a first preferred embodiment of the invention is designated as a whole with 1.

The support device 1 according to the present invention is apt to support a medical device above the body of a patient in a supine position on a surgical table during the execution of percutaneous interventional operations.

As shown in Figures 1 and 2, the support device 1 according to the preferred embodiment of the present invention comprises a main body substantially "squared C" shaped. The main body comprises a supporting surface 2 and upright elements 3.

Preferably, the main body has a substantially laminar structure thus presenting an inner face facing the patient body and an outer face apt to support medical devices. In particular, the main body is formed from a single laminar element.

The upright elements 3 are arranged on opposite sides of the support surface 2 and extend substantially orthogonal to the surface 2 itself.

Advantageously, the surface 2 - on which in a use configuration medical devices are placed - is a rigid, flat and wide surface in such a way as to allow an optimized handling of medical devices and to prevent unwanted damage due to the relative fragility of the devices.

Each upright element 3 has a supporting base 30 on the surgical table and is shaped to allow a spacing of the supporting surface 2 with respect to the surgical table during said operations.

As shown, the upright elements 3 further comprise adjusting means 7 apt to adjust the spacing of the supporting surface 2 with respect to the surgical table such as to allow an adaptation of said spacing to the specific physical conformation of the patient positioned or positionable between the supporting surface 2 and the surgical table.

In particular, the main body comprises an opening 20 at the supporting surface 2, shaped to allow the insertion of at least a portion of a foot of the patient.

The opening is dimensioned to allow the insertion of at least a portion, and in particular the portion comprising the fingers, of both feet of the patient. In the present example, as shown in Figures 1 and 2, two openings 20 are provided, each one dimensioned to contain one end of a patient foot. Advantageously, this configuration prevents unwanted rotations and/or movements of the patient feet and therefore prevents undesirable movements of the patient legs during surgery.

In an alternative embodiment of the invention, the realization of a single opening or a single central hole 20 shaped for the insertion of both ends of the patient feet is provided.

The adjusting means is shaped in such a way as to present a variation of the distance of the external profile by a chosen fixing point, depending on the specific angle considered.

In the present embodiment, the adjusting means 7 is "half-moon" shaped.

The adjusting means 7 is fixed to the upright elements 3 by means of detachable fixing means 9, 9', for example fixing screws or pins of known type.

Preferably, at a portion of the base of said "half-moon", each adjusting means has two openings apt to receive two screws or pins 9 and 9 ' to fix the adjusting means to the upright elements 3.

In particular, each upright element 3 includes at least one receiving seat 8 and/or 8' for receiving the aforesaid detachable fixing means 9 and/or 9'.

In the present example, in correspondence of each end of the supporting base 30, a first opening 8 for receiving first fixing means 9 and one or more further openings 8' for receiving second fixing means 9' are provided. Preferably, the further openings 8' are mutually spaced along a semicircle shaped pattern, in such a way that they have variable heights from the supporting base 30. Advantageously, it is therefore possible to adjust the height of the uprights 3 by spacing the supporting base 30 with respect to the surgical table by means of a rotation of the "half-moon" 7 around the first fixing pin 9.

Once the device 1 is positioned above the patient body, is possible to adjust the spacing of the supporting surface 2 with respect to the surgical table, depending on the specific physical conformation of the patient, so as to stabilize the positioning of the device 1 on the surgical table and at the same time so as to prevent the weight of the support device weighs on the patient.

Once the desired spacing is reached, the "half-moon" 7 is fixed at the ends of each upright element 3 by means of the second pin 9' at the further opening 8' desired.

Preferably, the adjusting means 7 is fixed in such a way as to obtain the same spacing in correspondence of each upright. Alternatively, it is provided to adjust each adjusting means 7 in a manner independent from the other ones.

In a further embodiment, not shown, the fixing of the adjusting means to the upright elements by means of a single fixing pin or screw is provided. For example, by rotating the "half-moon" around the axis of the pin it is possible to adjust the spacing of the base 30 with respect to the surgical table.

Advantageously, the device 1 also comprises a cranial surgical plane 6 apt to cover a portion of the patient body during the execution of an operation. Preferably, the contour of the cranial surgical plane has an ergonomic profile. For example, a portion 15 of the contour of the cranial surgical plane 6 facing the abdomen of the patient is "U" shaped in such a way as to increase the useful surface in contact with patient body and give more stability to the device 1 itself.

Preferably, a further portion 50 of the contour of the cranial surgical plane 6 facing towards patient feet is "double U" shaped, in such a manner as to allow a contact with the fore-part of the patient feet, in particular without interfering with the useful space provided at the apertures 20 for receiving the ends of the patient feet.

The cranial surgical plane 6 is slidable with respect to the main body in such a way as to be completely inserted in a position underlying the supporting surface 2 in a non-use configuration and instead to be easily extracted from this position in a use configuration of the device 1.

Preferably, the main body comprises a sliding guide 4, for example positioned along the extension on an inner face of the upright elements 3 facing towards the patient, for the sliding of the cranial surgical plane 6 with respect to the supporting surface 2.

In particular, the cranial surgical plane 6 can be made of a material shielding X-rays to reduce the radiological exposure of body parts of the operators currently poorly protectable with wearable devices, such as hands.

As shown in the Figures, the device 1 also comprises a caudal surgical plane 10 apt to support a medical device during the execution of a percutaneous interventional operation.

The main body includes a further sliding guide 40, not visible in figures, for example positioned along the extension of the upright elements on an inner face facing the patient, for the sliding of the caudal surgical plane 10 with respect to the supporting surface 2.

Advantageously, the caudal surgical plane 10 is slidable with respect to the main body in such a way as to be completely inserted in a position underlying the supporting surface 2 in a non-use configuration and to be easily extracted from this position in a use configuration of the device 1. In particular, a further upright element is provided, arranged along an end portion of the caudal surgical table 10, extending substantially orthogonal to the plane 10. Such further upright element is positioned in such a way as to support the plane 10 in a use configuration and to avoid undesired deformations of the plane 10 caused by the applied weight.

Preferably, the support device according to the present invention is made of plastic material with appropriate characteristics of strength and lightness. In particular, it is possible to realize the device according to the present invention at least partially in an X-ray shielding material for the protection from radiation of body districts of doctor who operates on it.

The device is predisposed to work with any cardiology or radiology work table, it is quickly installable and adjustable.

It will be appreciated that the support device according to the invention, comprising a plurality of interconnected parts, may also be provided in the form of an assembly kit.

The present invention has been sofar described with reference to preferred embodiments. It is to be meant that other embodiments may exist, belonging to the same inventive core, as defined by the protection scope of the here-below reported claims.

## Claims

1. A support device (1), apt to support a medical device above the body of a patient in a supine position on a surgical table during the execution of percutaneous interventional operations, comprising:
- a main body substantially "squared C" shaped, having a supporting surface (2) and upright elements (3) arranged on opposite sides of said supporting surface and extending substantially orthogonal to the supporting surface, wherein each upright element has a supporting base (30) on the surgical table and is shaped for spacing said supporting surface (2) with respect to the surgical table during said operations,
wherein said upright elements (3) further comprise adjusting means (7) apt to adjust the spacing of said supporting surface (2) with respect to the surgical table such as to allow an adaptation of said spacing to the specific physical conformation of the patient positioned or positionable between said supporting surface (2) and said surgical table, **characterized in that** said main body comprises at least one opening (20) at said supporting surface (2), said opening (20) being shaped to allow the insertion of at least a portion of a foot of the patient.

2. The support device (1) according to the preceding claim, wherein said main body has a substantially laminar structure.

3. The support device (1) according to any one of the preceding claims, wherein said adjusting means (7) are "half-moon" shaped.

4. The support device (1) according to any one of the preceding claims, comprising detachable fixing means (9, 9') of said adjusting means (7) to said upright elements (3).

5. The support device (1) according to the preceding claim, wherein said upright elements (3) comprise at least one receiving seat (8, 8') for receiving said detachable fixing means (9, 9').

6. The support device (1) according to any one of the preceding claims, further comprising a cranial surgical plane (6) apt to cover a portion of the patient body during the execution of an operation.

7. The support device (1) according to any one of the preceding claims, further comprising a caudal surgical plane (10) apt to support a medical device during the execution of an operation.

8. The support device (1) according to claim 6, wherein said main body comprises a sliding guide (4) for the sliding of said cranial surgical plane (6) with respect to said supporting surface (2).

9. The support device (1) according to claim 7, wherein said main body comprises a further sliding guide for the sliding of said caudal surgical plane (10) with respect to said supporting surface (2).

10. The support device (1) according to any one of the preceding claims, which is provided as an assembly kit.

11. The support device (1) according to any one of the preceding claims, which is at least partially realized in an X-ray shielding material.

## Patentansprüche

1. Unterstützungsvorrichtung (1), die geeignet ist eine medizinische Vorrichtung über dem Körper eines Patienten in einer Rückenlage auf einem Operationstisch während der Durchführung von perkutanen operativen Eingriffen zu unterstützen, die umfasst:
- einen im Wesentlichen quadratisch C-förmigen Grundkörper, der über eine Stützfläche (2) und aufrechte Elemente (3) verfügt, die an gegenüberliegenden Seiten der Stützfläche angeordnet sind und sich im Wesentlichen orthogonal zu der Stützfläche erstrecken, wobei jedes aufrechte Element über eine tragende Basis (30) auf dem Operationstisch verfügt und so geformt ist, dass die Stützfläche (2) gegenüber dem Operationstisch während den Operationen auf Abstand angeordnet ist,
wobei die aufrechten Elemente (3) weiterhin umfassen: Einstellmittel (7), die geeignet sind den Abstand der Stützfläche (2) gegenüber dem Operationstisch so einzustellen, dass es möglich ist den Abstand der spezifischen physischen Beschaffenheit des Patienten anzupassen, den zwischen der Stützfläche (2) und dem Operationstisch platziert ist oder platziert werden kann, **dadurch gekennzeichnet, dass**
der Grundkörper mindestens eine Öffnung (20) in der Stützfläche (2) umfasst, wobei die Öffnung (20) so geformt ist, dass die Einführung mindestens eines Teils eines Fußes des Patienten möglich ist.

2. Unterstützungsvorrichtung (1) gemäß dem vorangehenden Anspruch, wobei der Grundkörper über eine im Wesentlichen laminare Struktur verfügt.

3. Unterstützungsvorrichtung (1) gemäß einem der vorangehenden Ansprüche, wobei die Einstellmittel (7) "halbmondförmig" sind.

4. Unterstützungsvorrichtung (1) gemäß einem der vorangehenden Ansprüche, die abnehmbare Befestigungselemente (9, 9') des Einstellmittels (7) an den aufrechten Elementen (3) umfasst.

5. Unterstützungsvorrichtung (1) gemäß dem vorangehenden Anspruch, wobei die aufrechten Elemente (3) mindestens einen Empfangsplatz (8, 8') zum Empfangen des abnehmbaren Befestigungselements (9, 9') umfassen.

6. Unterstützungsvorrichtung (1) gemäß einem der vorangehenden Ansprüche, die weiterhin eine kraniale Operationsfläche (6) umfasst, die geeignet ist einen Teil des Patientenkörpers während der Durchführung einer Operation zu bedecken.

7. Unterstützungsvorrichtung (1) gemäß einem der vorangehenden Ansprüche, die weiterhin eine kaudale Operationsfläche (10) umfasst, die geeignet ist während der Durchführung einer Operation eine medizinische Vorrichtung zu unterstützen.

8. Unterstützungsvorrichtung (1) gemäß Anspruch 6, wobei der Grundkörper eine Gleitführung (4) zum Gleiten der kranialen Operationsfläche (6) gegenüber der Stützfläche (2) umfasst.

9. Unterstützungsvorrichtung (1) gemäß Anspruch 7, wobei der Grundkörper eine weitere Gleitführung zum Gleiten der kaudalen Operationsfläche (10) gegenüber der Stützfläche (2) umfasst.

10. Unterstützungsvorrichtung (1) gemäß einem der vorangehenden Ansprüche, die als ein Bausatz zur Verfügung gestellt wird.

11. Unterstützungsvorrichtung (1) gemäß einem der vorangehenden Ansprüche, die mindestens zum Teil aus einem Röntgenschutzmaterial gefertigt ist.

## Revendications

1. Dispositif de support (1), apte à supporter un dispositif médical au-dessus du corps d'un patient qui est allongé sur le dos sur une table d'opération pendant l'exécution d'opérations percutanées, comprenant :
- un corps principal sensiblement en forme de « C carré », ayant une surface de support (2) et des éléments droits (3) agencés sur des côtés opposés de ladite surface de support et s'étendant sensiblement orthogonalement vers la surface de support, dans lequel chaque élément droit a une base de support (30) sur la table d'opération et est formé pour espacer ladite surface de support (2) par rapport à la table d'opération pendant lesdites opérations,
dans lequel lesdits éléments droits (3) comprennent en outre des moyens d'ajustement (7) aptes à ajuster l'espacement de ladite surface de support (2) par rapport à la table d'opération afin de permettre une adaptation dudit espacement à la conformation physique spécifique du patient positionné ou positionnable entre ladite surface de support (2) et ladite table d'opération,
**caractérisé en ce que** :
ledit corps principal comprend au moins une ouverture (20) au niveau de ladite surface de support (2), ladite ouverture (20) étant formée pour permettre l'insertion d'au moins une partie d'un pied du patient.

2. Dispositif de support (1) selon la revendication précédente, dans lequel ledit corps principal a une structure sensiblement laminaire.

3. Dispositif de support (1) selon l'une quelconque des revendications précédentes, dans lequel lesdits moyens d'ajustement (7) sont de forme de « demi-lune ».

4. Dispositif de support (1) selon l'une quelconque des revendications précédentes, comprenant des moyens de fixation détachables (9, 9') desdits moyens d'ajustement (7) sur lesdits éléments droits (3).

5. Dispositif de support (1) selon la revendication précédente, dans lequel lesdits éléments droits (3) comprennent au moins un siège de réception (8, 8') pour recevoir lesdits moyens de fixation détachables (9, 9').

6. Dispositif de support (1) selon l'une quelconque des revendications précédentes, comprenant en outre un plan chirurgical crânien (6) apte à couvrir une partie du corps du patient pendant l'exécution d'une opération.

7. Dispositif de support (1) selon l'une quelconque des revendications précédentes, comprenant en outre un plan chirurgical caudal (10) apte à supporter un dispositif médical pendant l'exécution d'une opération.

8. Dispositif de support (1) selon la revendication 6, dans lequel ledit corps principal comprend un guide de coulissement (4) pour le coulissement dudit plan chirurgical crânien (6) par rapport à ladite surface de support (2).

9. Dispositif de support (1) selon la revendication 7, dans lequel ledit corps principal comprend un autre guide de coulissement pour le coulissement dudit plan chirurgical caudal (10) par rapport à ladite surface de support (2).

10. Dispositif de support (1) selon l'une quelconque des revendications précédentes, qui est fourni sous la forme d'un kit d'assemblage.

11. Dispositif de support (1) selon l'une quelconque des revendications précédentes, qui est au moins partiellement réalisé avec un matériau de protection contre les rayons X.
